# EUROPEAN PATENT APPLICATION

(11) **EP 1 738 703 A1**
(43) Date of publication of application: **03.01.2007**
(21) Application number: 04821907.5
(22) Date of filing: 12.11.2004
(51) Int. Cl.: A61B 18/00

(54) **ULTRASONIC TREATMENT TOOL**

(30) Priority: 20.04.2004 JP 2004124184
(71) Applicant: Olympus Corporation, Tokyo 151-0072 (JP)
(72) Inventor: YAMADA, Norihiro, Hachioji-shi, Tokyo 1920045 (JP)
(74) Representative: von Hellfeld, Axel
(86) International application number: PCT/JP2004/016877
(87) International publication number: WO 2005/102197

(57) **Abstract**

An ultrasonic treatment apparatus (1) for transmitting ultrasonic vibration generated by an ultrasonic vibration generating unit (2) to a head of a transmission member (3) having flexibility and for giving ultrasonic treatment, includes a treatment portion (3a) provided at the head of the transmission member (3), and a grasping member (5) which is provided to the treatment portion (3a), and opened/closed by an operation wire (6) to grasp body tissue in engagement with the treatment portion (3a). The treatment portion (3a) has a length which is approximately 1/2 of a wavelength of the ultrasonic vibration, the head of the treatment portion (3a) is set to be an antinode of an ultrasonic vibration, has larger cross section area on a surface orthogonal to a transmission direction of the ultrasonic vibration than the transmission member (3), and is provided with more stiffness than the transmission member (3). The grasping member (5) is engaged with the treatment portion (3a) at a node position of the ultrasonic vibration. The operation wire (6) is connected to the grasping member (5) at one end, and to the operation member (9) at the other end, so that the operation wire (6) is handled by the operation member (9).

## Description

### TECHNICAL FIELD

The present invention relates to an ultrasonic treatment apparatus.

### BACKGROUND ART

Conventionally, an ultrasonic treatment apparatus has widely been used for incision (resection) of body tissue as well as coagulation of the body part where incision treatment or the like is applied. Such an ultrasonic treatment apparatus, for example, includes a treatment portion having a loop shape or a stick shape at the distal end of a probe for transmitting ultrasonic vibration, to give ultrasonic treatment to body tissue (see Patent Documents 1 and 2, for example).

Patent Document 1: United States Patent No. 6231578
Patent Document 2: United States Patent No. 5649935

### DISCLOSURE OF INVENTION

### PROBLEM TO BE SOLVED BY THE INVENTION

In a so-called flexible endoscope having a bendable insert part to be inserted into a body cavity, an ultrasonic treatment apparatus using a flexible probe which can be bent into a curvature of the insert part is employed. Here, the flexible probe has a property of returning substantially to an original form via an elastic deformation when bent at 90 degrees or more, for example. A conventional ultrasonic treatment apparatus using such a flexible probe, which does not include a forceps at the distal end of the probe, cannot perform ultrasonic treatment with body tissue grasped, and thereby an ultrasonic treatment having such a function has been desired.

In view of the foregoing, an object of the present invention is to provide an ultrasonic treatment apparatus using a flexible probe which allows ultrasonic treatment with body tissue grasped.

### MEANS FOR SOLVING PROBLEM

To solve the above problems and achieve the object, an ultrasonic treatment apparatus according to claim 1 transmits ultrasonic vibration generated by an ultrasonic generating unit to a distal end of a transmission member having flexibility to perform ultrasonic treatment, includes a treatment portion provided at the distal end of the transmission member, and a grasping member which is opened or closed via an operation wire for grasping body tissue in engagement with the treatment portion. The treatment portion has a length which is approximately 1/2 of a wavelength of the ultrasonic vibration, and the distal end of the treatment portion is located at an ultrasonic vibration antinode.
The treatment portion has a cross section area on a plane orthogonal to a transmission direction of the ultrasonic vibration, and the treatment portion has a stiffness in which the cross section area is larger than a cross section area of the treatment member. The grasping member is engaged with the treatment portion at a node position of the ultrasonic vibration. The operation wire is connected to the grasping member at one end, and to the operation member at the other end, and handled by the operation member.

In the ultrasonic treatment apparatus according to claim 1, a forceps and the treatment portion provided with stiffness grasp body tissue to give ultrasonic treatment to the body tissue by the ultrasonic vibration transmitted via the flexible transmission member.

In the ultrasonic treatment apparatus according to claim 2, the transmission member is covered with a sheath.

In the ultrasonic treatment apparatus according to claim 3, the transmission member is provided with a damping member, which is located at an ultrasonic vibration node and in direct contact with the inside of the sheath.

### EFFECT OF THE INVENTION

The ultrasonic treatment apparatus according to the present invention, which includes the treatment portion provided with stiffness, is advantageous in that ultrasonic treatment can be performed with body tissue grasped by the grasping member and the treatment portion.

### BRIEF DESCRIPTION OF DRAWINGS

FIG. 1 is a cross-sectional view, partly broken away, of schematic structure of an ultrasonic treatment apparatus according to the present invention;
FIG. 2 is a perspective view showing a treatment portion, a forceps, and an operation wire of the ultrasonic treatment apparatus shown in FIG. 1;
FIG. 3 is a bottom view, in partial section, of FIG. 2;
FIG. 4A is a cross-sectional view of a first modification in which the forceps supports the treatment portion with flexible opening and closing;
FIG. 4B is a cross-sectional view of a second modification in which the forceps supports the treatment portion with flexible opening and closing;
FIG. 5 is a cross-sectional view of a substantial part showing a modification of the ultrasonic treatment apparatus shown in FIG. 1;
FIG. 6 shows one example of methods of attaching a damping member to a flexible probe;
FIG. 7 is a perspective view, showing a concavity shape of the flexible probe to which the damping member is attached, of another example of methods of attaching the damping member to the flexible probe; and
FIG. 8 is a perspective view showing still another example of methods of attaching the damping member to the flexible probe.

### EXPLANATIONS OF LETTERS OR NUMERALS

- 1: Ultrasonic treatment apparatus
- 2: Ultrasonic transducer unit
- 3: Flexible probe
- 3a: Treatment portion
- 3b: Transition portion
- 4: Covering member
- 4a: Attachment surface
- 4b: Cylindrical portion
- 4c: Opening
- 5: Forceps
- 5c: Pin
- 6: Operation wire
- 7: Sheath
- 8: Supporting member
- 9: Operation unit
- 9a: Supporting tube
- 9b: Guiding member
- 9c: Movable handle
- 9d: Fixed handle
- 9e, 9f: Finger hooking holes
- 11: Inner sheath
- 12, 13: Damping members

### BEST MODE(S) FOR CARRYING OUT THE INVENTION

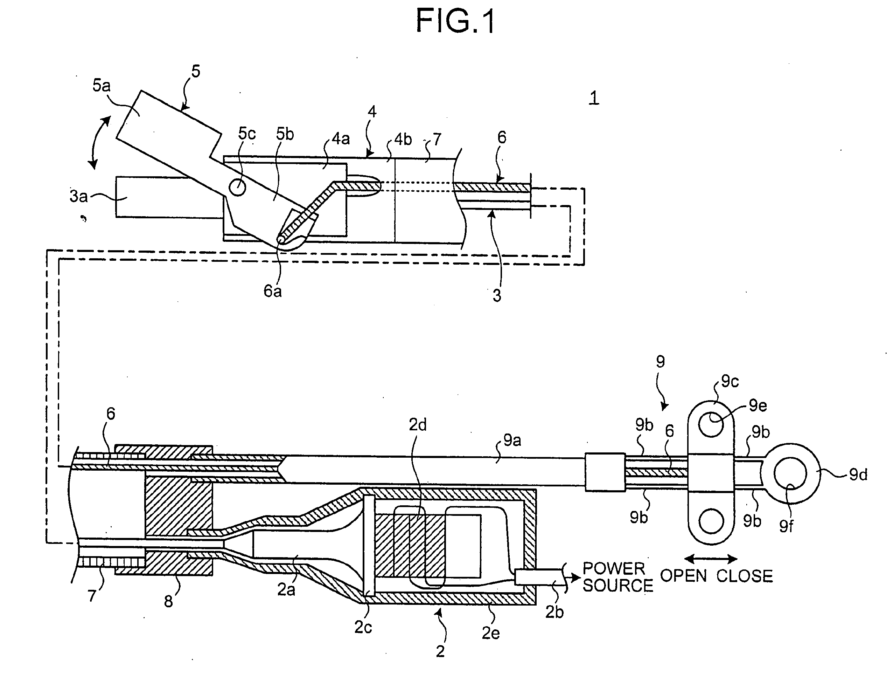

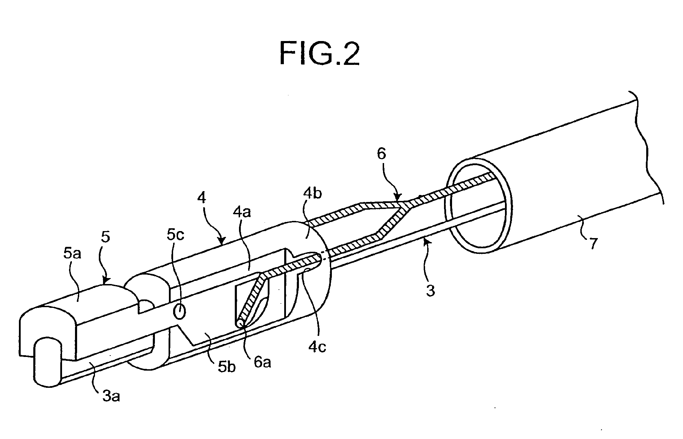

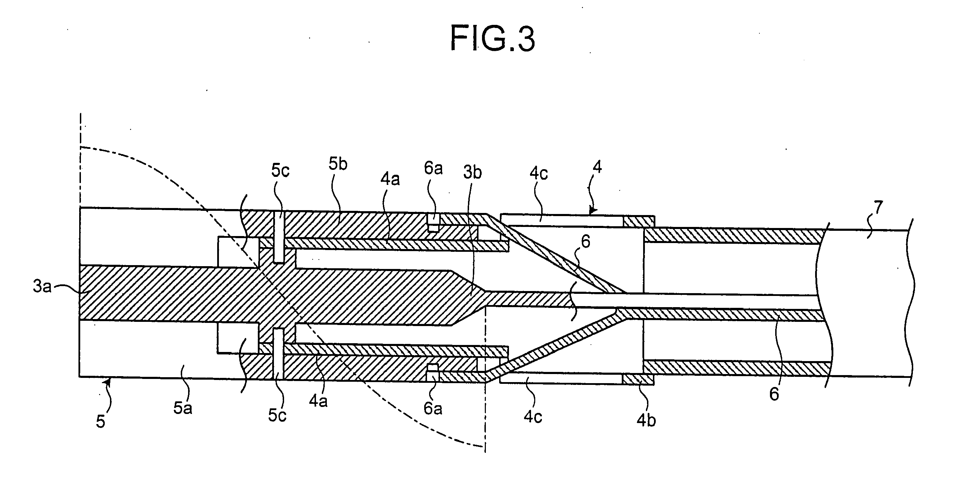

Exemplary embodiments of the ultrasonic treatment apparatus according to the present invention will be described in detail with reference to the accompanying drawings. FIG. 1 is a cross-sectional view, partly broken away, of schematic structure of an ultrasonic treatment apparatus according to the present invention. FIG. 2 is a perspective view showing a treatment portion, a forceps, and an operation wire of the ultrasonic treatment apparatus shown in FIG. 1. FIG. 3 is a bottom view, in partial section, of FIG. 2.

An ultrasonic treatment apparatus 1, as shown in FIG. 1, includes an ultrasonic transducer unit 2, a long flexible probe 3, a forceps 5, and an operation unit 9.

The ultrasonic transducer 2 is a bolted Langevin transducer having a horn 2a as shown in FIG. 1, attached to a supporting member 8, and connected to a power source with a power source cable 2b. In the ultrasonic transducer unit 2, an ultrasonic transducer 2d and the horn 2a are fixed to a casing 2e on contact with a flange 2c. Here, the ultrasonic treatment apparatus 1 is switched on/off by a foot switch provided between the power source cable 2b and the power source.

The flexible probe 3 connected to the ultrasonic transducer 2d at one end thereof as shown in FIG. 1, is a transmission member having enough flexibility to transmit ultrasonic vibration generated by the ultrasonic transducer 2d, is made of, for example, titanium alloy, nickel alloy, stainless steel, duralumin, or silica optical fiber. The flexible probe 3 is provided with a treatment portion 3a at the other end. The flexible probe 3 has enough flexibility to return substantially to an original form via an elastic deformation when bent at 90 degrees or more, for example.

The treatment portion 3a, which is provided at the distal end of the flexible probe 3 via a tapered transition portion 3b as shown in FIGS. 2 and 3, has a length which is approximately λ/2 of the wavelength (λ) of the ultrasonic vibration generated by the ultrasonic transducer 2d (see the dotted line in FIG. 3), and the distal end of the treatment portion 3a is set to be located at an ultrasonic vibration antinode. The treatment portion 3a has a cross section area, on the plane orthogonal to a transmission direction of the ultrasonic vibration, larger than that of the flexible probe 3, so that the treatment portion 3a has stiffness more than that of the flexible probe 3. A ratio of the cross section area of the treatment portion 3a to that of the flexible probe 3 (=St/Sp) is, for example, set in a range expressed as 1.3 ≤ St/Sp ≤ 8. Moreover the treatment portion 3a is inserted into a covering member 4, and the distal end of the treatment portion 3a protrudes out of the covering member 4. Since the cross section area of the treatment portion 3a is larger than that of the flexible probe 3, the treatment portion 3a is provided with enough stiffness not to yield to a pressure in grasping body tissue with the forceps 5.

When the cross section area of the treatment portion 3a is set to be larger than that of the flexible probe 3, the closer the transition portion 3b is located at an ultrasonic vibration node, the larger the damping rate of vibration amplitude becomes. Hence, as shown in FIG. 3, the transition portion 3b is provided at an ultrasonic vibration antinode (see the dotted line in FIG. 3), so that the damping rate of vibration amplitude is suppressed to the minimum. Though the cross section area of the treatment portion 3a is larger than that of the flexible probe 3, the ratio of the cross section area of the treatment portion 3a to that of the flexible probe 3 (Sp) is as small as 1.3 ≤ St/Sp ≤ 8, compared with a general value (10 to 30) in regard to the ratio of the cross section area of the ultrasonic transducer 2d to that of the flexible probe 3. Thus, the flexible probe 3 even with the treatment portion 3a is not greatly affected in the damping rate of vibration amplitude. The treatment portion 3a is provided with the forceps 5 which is flexibly opened and closed to grasp body tissue in engagement with the treatment portion 3a at an ultrasonic vibration node.

The covering member 4 has a substantially cylindrical shape; attachment surfaces 4a are formed on both sides of the distal end which are in parallel with each other for attaching the forceps 5; and openings 4c through which an operation wire 6 is pulled to the attachment surface 4a are formed in a cylindrical portion 4b formed at the rear part, as shown in FIGS. 2 and 3. The cylindrical portion 4b of the covering member 4 is connected to one end of a sheath 7. The sheath 7 is connected to the supporting member 8 at the other end, covering the flexible probe 3 and the operation wire 6.

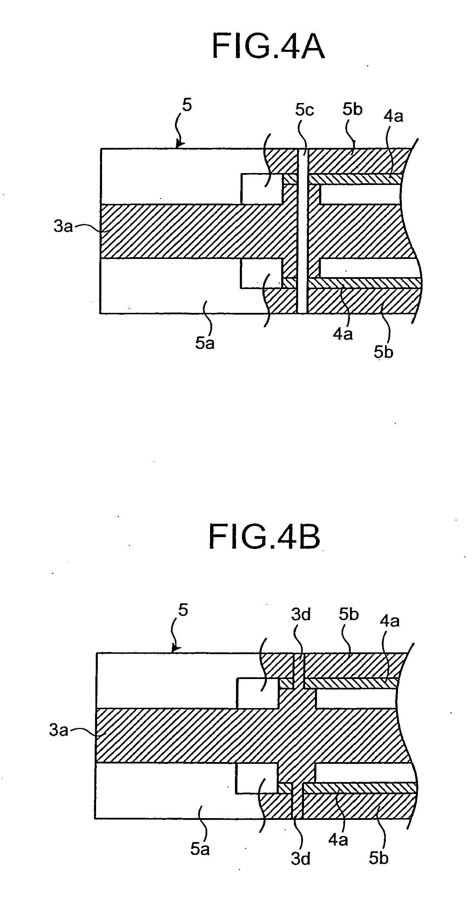

The operation unit 9 controls opening and closing of the forceps 5 about a pin 5c with the operation wire 6 which is connected to the forceps 5 at one end and to the operation unit 9 at the other end (see the arrow shown in FIG. 1). The forceps 5 has connection arms 5b which extend backward from both sides of a main body 5a engaging with the treatment portion 3a. Two connection arms 5b of the forceps 5 are in direct contact with the attachment surface 4a and connected to the treatment unit 3a with the pin 5c attached at an ultrasonic vibration node(see FIG. 3). The forceps 5, which is connected to the treatment portion 3a at an ultrasonic vibration node, is not affected by the ultrasonic vibration of the treatment portion 3a. The pin 5c may be attached to penetrate through the treatment portion 3a as shown in FIG. 4A; besides, instead of the pin 5c, a protrusion 3d provided at an ultrasonic vibration node of the treatment portion 3a may support the forceps 5 to the treatment portion 3a to enable opening and closing of the forceps 5.

The operation wire 6 branches into two at one end as shown in FIG. 2, the branched two wires are connected to wire pins 6a attached rearward of the two connection arms 5b, respectively. The operation wire 6 is connected to a movable handle 9c of the operation unit 9 at the other end.

The operation unit 9 includes a supporting tube 9a attached to the supporting member 8, two guiding members 9b, the movable handle 9c, and a fixed handle 9d, as shown in FIG. 1. The supporting tube 9a guides the operation wire 6 inserted inside to the movable handle 9c. The guiding members 9b slidably guide the movable handle 9c along the operation wire 6. The movable handle 9c is a plate-like member to which the operation wire 6 is connected, and has finger hooking holes 9e formed, respectively in both ends thereof. When the movable handle 9c is shifted toward the arrow direction along the guiding members 9b in FIG. 1, the forceps 5 is opened or closed via the operation wire 6. The fixed handle 9d is attached to rear end of the guiding members 9b, and has a finger hooking hole 9f.

The ultrasonic treatment apparatus 1 having such a structure, when used, is inserted into a channel of an endoscope so that the treatment portion 3a and the forceps 5 protrude from the distal end of the endoscope, or is inserted directly into a body cavity. When the movable handle 9c is pulled to the fixed handle 9d, and the forceps 5 is turned anti-clockwise about the pin 5c via the operation wire 6 in the ultrasonic treatment apparatus 1 in FIG. 1, the treatment portion 3a engaged with the forceps 5 in a closed state is inserted into the channel or the like.

The movable handle 9c is pushed and pulled away from the fixed handle 9d after the insertion, and thereby the forceps 5 is turned clockwise about the pin 5c via the operation wire 6 and becomes in an open state where the engagement with the treatment portion 3a is released. While the body cavity is observed by the endoscope, the forceps 5 is again closed by the operation of the movable handle 9c to grasp the body tissue at a desired part between the treatment portion 3a and the forceps 5 for ultrasonic treatment. Here, the treatment portion 3a has a cross section area larger than that of the flexible probe 3, thereby having high stiffness. Hence, though the flexible probe 3 has a flexible property in the ultrasonic treatment apparatus 1, the treatment portion 3a with high stiffness and the forceps 5 surely grasp the body tissue for ultrasonic treatment.

In the ultrasonic treatment apparatus 1, the treatment portion 3a has a length which is substantially 1/2 of the wavelength of the ultrasonic vibration transmitted via the flexible probe 3, and the distal end of the treatment portion 3a is set to be located at an antinode of the ultrasonic vibration. Hence, in grasping body tissue with the treatment portion 3a and the forceps 5, the ultrasonic treatment apparatus 1 can provide the most effective ultrasonic treatment on the grasped body tissue.

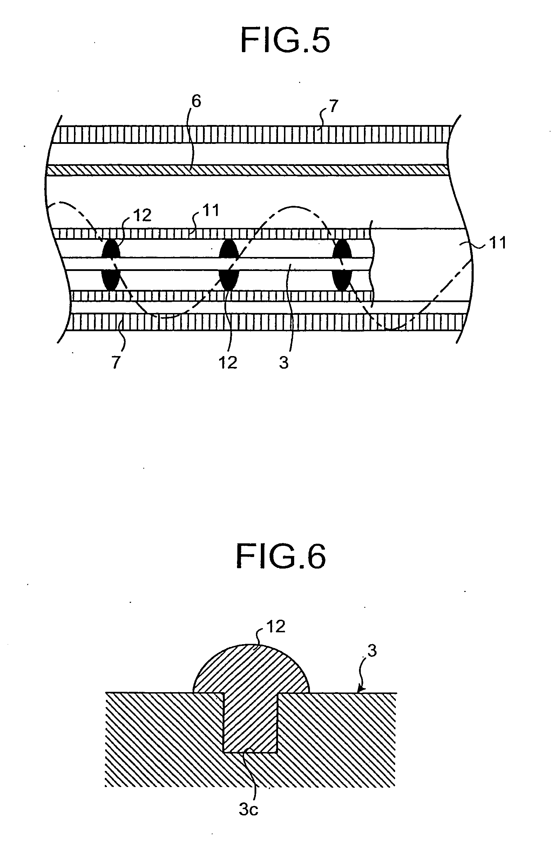

In the ultrasonic treatment apparatus 1 as shown in FIG. 5, the flexible probe 3 may be covered with an inner sheath 11, and a damping member 12 which is in direct contact with the inside of the inner sheath 11 may be provided at an ultrasonic vibration node of the flexible probe 3 (see the dotted line). When the flexible probe 3 is covered with the inner sheath 11 in the ultrasonic treatment apparatus 1, the inner sheath 11 separates the flexible probe 3 from the operation wire 6 that moves in the horizontal direction in FIG. 5 according to an opening and closing operation of the forceps 5, and thereby prevents interference and entanglement between the flexible probe 3 and the operation wire 6 inside the sheath 7.

When the flexible probe 3 is provided with the damping member 12, a noise vibration of the flexible probe 3 caused by the vibration of the ultrasonic vibration transmitted via the flexible probe 3 is suppressed, resulting in a stable transmission of the ultrasonic vibration. Here the damping member 12 is made of material such as fluorocarbon polymer having thermostability, polyimide, rubber, and the like, and is attached to a concavity 3c formed along the circumferential direction on the flexible probe 3, as shown in FIG. 6.

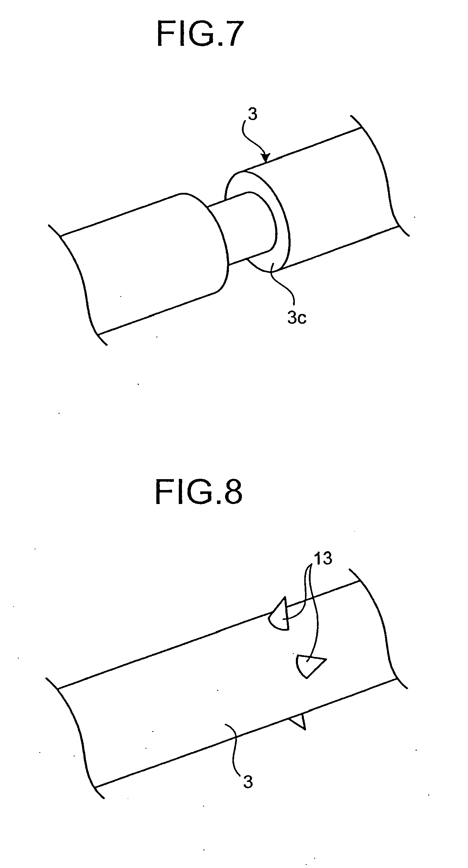

The concavity 3c formed along the circumferential direction on the flexible probe 3 may be a groove extending along a longitudinal direction of the flexible probe 3, as shown in FIG. 7; the damping member 12 may be attached to the groove, and thereby provided over several millimeters of range around an ultrasonic vibration node. Moreover, a damping member may be spaced out in a circumferential direction on outer circumference of the flexible probe 3, like a damping member 13 shown in FIG. 8.

### INDUSTRIAL APPLICABILITY

The ultrasonic treatment apparatus according to the present invention includes a treatment portion with stiffness, and thereby is useful as an ultrasonic treatment apparatus using a flexible probe.

## Claims

1. An ultrasonic treatment apparatus for transmitting ultrasonic vibration generated by an ultrasonic vibration generating unit to a distal end of a transmission member having flexibility to perform ultrasonic treatment, comprising:
a treatment portion provided at the distal end of the transmission member; and
a grasping member which is provided to the treatment portion, and opened or closed by an operation wire to grasp body tissue in engagement with the treatment portion, wherein
the treatment portion has a length which is approximately 1/2 of a wavelength of the ultrasonic vibration, and has a cross section area on a plane orthogonal to a transmission direction of the ultrasonic vibration, and has a stiffness in which the cross section area is larger than a cross section area of the transmission member,
the distal end of the treatment portion is located at an ultrasonic vibration antinode,
the grasping member is engaged with the treatment portion at the ultrasonic vibration node, and
the operation wire is connected to the grasping member at one end, and to the operation member at the other end, so that the operation wire is handled by the operation member.

2. The ultrasonic treatment apparatus according to claim 1, wherein the transmission member is covered with a sheath.

3. The ultrasonic treatment apparatus according to claim 1 or 2, wherein the transmission member is provided with a damping member which is located at an ultrasonic vibration node and in direct contact with an inside of the sheath.
